# EUROPÄISCHE PATENTSCHRIFT

(11) **EP 2 099 462 B1**
(45) Veröffentlichungstag und Bekanntmachung des Hinweises auf die Patenterteilung: **08.06.2011**
(21) Anmeldenummer: 07818284.7
(22) Anmeldetag: 20.09.2007
(51) Int. Cl.: A61K 31/728, A61K 31/737, A61P 13/10

(54) **PHARMAZEUTISCHE ZUBEREITUNG FÜR DIE BEHANDLUNG ENTZÜNDLICHER ERKRANKUNGEN DES UROGENITALTRAKTES**
PHARMACEUTICAL PREPARATION FOR THE TREATMENT OF INFLAMMATORY DISEASES OF THE UROGENITAL TRACT
PRÉPARATION PHARMACEUTIQUE POUR LE TRAITEMENT DE PATHOLOGIES INFLAMMATOIRES DU SYSTÈME UROGÉNITAL

(30) Priorität: 12.12.2006 DE 102006058776; 20.12.2006 DE 102006060953
(43) Veröffentlichungstag der Anmeldung: 16.09.2009
(73) Patentinhaber: Farco-Pharma GmbH, 50670 Köln (DE)
(72) Erfinder: VESTWEBER, Anna-Maria, 51399 Burscheid (DE)
(74) Vertreter: Gesthuysen, Michael
(86) Internationale Anmeldenummer: PCT/EP2007/008194
(87) Internationale Veröffentlichungsnummer: WO 2008/071245

(56) Entgegenhaltungen:
- WO-A-02/09728
- WO-A-96/25168
- US-A- 5 880 108
- US-A1- 2003 175 332
- US-A1- 2004 161 476
- US-A1- 2006 234 978

## Beschreibung

Die vorliegende Erfindung betrifft eine Zubereitung Zusammensetzung, insbesondere eine pharmazeutische Zubereitung, vorzugsweise zur Behandlung von entzündlichen Erkrankungen des Urogenitaltraktes, vorzugsweise von interstitieller Cystitis. Die Zubereitung nach der Erfindung enthält in Kombination Hyaluronsäure bzw. deren Salze, vorzugsweise mit einer mittleren Molmasse (Molekulargewicht) von weniger als 200 kDa mit mindestens einem weiteren, hiervon verschiedene Glykosaminoglykan, vorzugsweise Chondroitinsulfat.

Des weiteren betrifft die vorliegende Erfindung ein Behältnis und eine Applikationsvorrichtung zur Instillation und/oder Applikation in den Urogenitalbereich, welche jeweils die erfindungsgemäße Zubereitung enthalten.

Schließlich betrifft die vorliegende Erfindung die Verwendung der Zubereitung ach der Erfindung zur Behandlung von Erkrankungen des Urogenitaltraktes, wobei es sich hierbei insbesondere um interstitielle Cystitis handelt.

Das Krankheitsbild der Cystitis kann im allgemeinen in zwei Gruppen eingeteilt werden. Neben einer solchen Cystitis, die durch insbesondere bakterielle Infektionen verursacht wird und die in der Regel mit Antibiotika oder aber durch chirurgische Intervention zur Beseitigung von Ursachen der Infektion, z. B. bei Obstruktionen oder Reflux, zu therapieren ist, gibt es eine Reihe von Entzündungen der Blase, die nicht durch Infektionen hervorgerufen werden. Dazu zählen die radiogene Cystitis (= Strahlencystitis) und die interstitielle Cystitis.

Die radiogene Cystitis tritt bei ca. 5 % der wegen Malignomen im kleinen Becken bestrahlten Patienten auf. Diese hämorrhagische Cystitis tritt in der Regel sechs Monate bis zehn Jahre nach der Bestrahlung auf und kann auf wahrscheinlich irreversible Gewebeveränderungen zurückgeführt werden.

Die Behandlungsmöglichkeiten bei der radiogenen Cystitis umfassen eine Therapie mit Antispasmodika, z. B. Trospiumchlorid, Darifenacin etc. oder eine sogenannte hyperbare Oxygenierung.

Ein weitaus größeres Feld ist die interstitielle Cystitis, insbesondere da hiervon ein großer Personenkreis betroffen ist. Die interstitielle Cystitis wird mitunter auch als "chronisch idiopathische Blasenentzündung unklarer Genese" definiert. Die interstitielle Cystitis ist schwer zu diagnostizieren, und ihre Behandlungsmöglichkeiten werden als schwierig angesehen. Die interstitielle Cystitis wird auch unter den Ausdruck "*painful bladder syndrome*" (schmerzhaftes Blasensyndrom) subsumiert.

Hinsichtlich der Ätiologie gibt es mitunter noch keine vollständig gesicherten Erkenntnisse. Diesbezüglich werden diverse Hypothesen diskutiert, wie eine Freisetzung von inflammatorischen Substanzen durch Mastzellaktivierung aufgrund von unterschiedlichen Stimuli; okkulte Infektionen; Steigerung der Durchlässigkeit der Blasenwand für toxische Substanzen; immunologische Prozesse sowie eine Hypersensitivität von Nervenfasern mit Erhöhung der Nervenfaserdichte.

Das diagnostische Spektrum der interstitielle Cystitis umfaßt neben dem Miktionsprotokoll und dem Schmerztagebuch mit VAS sowie der bakteriologischen Untersuchung zum Ausschluß eines Harnwegsinfektes und der Urinzystologie zum Ausschluß eines Karzinoms in situ und die unter Narkose durchzuführende Cystoskopie mit gegebenenfalls einhergehender Biopsie.

Die interstitielle Cystitis ist eine chronisch-entzündliche Erkrankung der Blase ohne nachweisbare Bakterien im Urin. Folglich handelt es sich hierbei um eine Cystitis nichtbakteriellen Ursprungs. Es handelt sich um eine bis heute nicht völlig geklärte Erkrankung, unter der die Patienten oft schlimmer leiden als unter einer Tumorerkrankung. Auch das *US National Health Institute* hat die interstitielle Cystitis als Erkrankung mit höherer Priorität eingestuft. Die Lebensqualität kann durch starken Harndrang, häufiges Wasserlassen tagsüber wie auch in der Nacht und zunehmenden Schmerzen extrem beeinträchtigt werden.

Die Häufigkeit der interstitiellen Cystitis wird mit 53 bis 67 Fällen auf 100.000 Menschen in den Vereinigten Staaten von Amerika und in Europa mit 18 Fällen auf 100.000 Menschen in der Bevölkerung angegeben. Die Abweichung erklärt sich mitunter aus dem unterschiedlich ausgeprägten Wissen um die Erkrankung und deren Diagnostik. Nach jüngsten Erhebungen scheint die Erkrankung zudem weiter zuzunehmen. Patienten, bei denen die Erkrankung auftritt, sind meist im mittleren Lebensalter. Die Erkrankung kommt im Verhältnis bei Frauen siebenmal häufiger vor als bei Männern. Ein Grund dafür kann in der Anatomie der Frau gesehen werden, deren Harnröhre kürzer ist als die des Mannes. Dies führt zu einer höheren Anfälligkeit gegenüber aufsteigenden Harnwegsinfektionen. Bei Frauen mit wiederholten (rezidivierenden) Harnwegsinfektionen ist die Schleimhaut der Blase in stärkerem Maße geschädigt. Diese ständige Reizung kann zu der nichtbakteriellen, insbesondere chronischen interstitiellen Cystitis führen. Die interstitielle Cystitis wird somit überwiegend bei Frauen diagnostiziert. Die Epidemiologie der interstitiellen Cystitis ist schwer einzuschätzen und ist mitunter sehr unterschiedlich. So wird die Prävalenz in Finnland mit nur 1,2 bis 1,6 Frauen angegeben.

Die häufigsten Symptome der interstitiellen Cystitis sind (Reihenfolge in absteigender Häufigkeit);
- erhöhte Harndrang;
- häufiges Wasserlassen;
- Schmerzen des Beckens, des unteren Bauchraumes und des Dammes;
- Beckendruck;
- Schmerzen beim Wasserlassen, verbunden mit Abgabe geringster Mengen von Urin;
- starker Schmerz während und nach dem Geschlechtsverkehr ;
- brennendes Schmerzgefühl;
- massive Durchschlafprobleme durch Schmerzen;
- Blut im Urin.

Die Hauptsymptome der interstitiellen Cystitis sind somit ein Kapazitätsverlust der Blase mit füllungsabhängigen Schmerzen und häufigem und massivem Harndrang. Man spricht auch von der Trias: "*frequency, urgency, pain*" (Häugfigkeit, Dringlichkeit, Schmerzen).

Die vorgenannten Symptome der interstitiellen Cystitis können z. B. durch eine verstärkte Schmerzempfindlichkeit oder durch psychische Faktoren noch zunehmen. Gezeigt wurde eine Störung der Zusammensetzung der Glykosaminoglykanschicht, und immunhistochemische Untersuchungen zeigen u. a. eine verminderte Chondroitin-4-sulfat-Färbung.

Ohne sich auf eine Theorie festlegen zu wollen, kann die Entstehung und Entwicklung der interstitiellen Cystitis wie folgt erklärt werden: Durch Lükken der Schurtzschicht der Blasenschleimhaut, der sogenannten Glykosaminoglykanschicht, gelangen Bakterien, Mikrokristalle, Proteine und/oder schädliche, gelöste Bestandteile des Harns, z. B. Harnstoff, direkt in tiefere Schichten der Blasenschleimhaut und bewirken dort eine weitere Schädigung.

Durch die Schädigung der Blasenschleimhaut und durch die resultierende chronische Entzündung kommt es zu Reparaturvorgängen, die oftmals mit einer Vernarbung einhergehen. Dies kann zu einer eingeschränkten Elastizität der Harnblasenwand und zu einem zunehmenden Verlust des Fassungsvermögens der Harnblase führen. Im Spätstadium der interstitiellen Cystitis kann eine Schrumpfblase entstehen, und eine operative Entfernung der Harnblase kann unter bestimmten Umständen notwendig werden. Daher ist eine frühzeitige Erkennung und Therapie zur Vermeidung des Spätstadiums der interstitiellen Cystitis von großer Bedeutung.

Die Hauptursache der interstitiellen Cystitis ist die Schädigung der Blasenschleimhaut. Die Blasenschleimhaut ist durch eine Schutzschicht, die unter anderem Hyaluronsäure enthält, gegen Mikroorganismen, krebsverursachende Substanzen, und andere schädliche Stoffe, die im Urin vorkommen, abgeschirmt. Diese Schutzschicht ist extrem wasserbindend und bildet sozusagen einen "Wasserfilm" und somit eine weitere physikalische Barriere gegen schädigende Substanzen im Urin.

Bei Patienten mit interstitieller Cystitis bestehen Defekte in dieser Schutzschicht der Blasenschleimhaut. Insbesondere hat man einen Verlust von Hyaluronsäure festgestellt.

Weitere Ursachen der interstitiellen Cystitis können z. B. Autoimmunreaktionen, die sich gegen körpereigene Zellen in der Blase richten, oder frühere chronische bakterielle Infektionen sein.

Die typische Symptomatik der interstitiellen Cystitis ist häufiges Wasserlassen, verstärkter Harndrang sowie in manchen Fällen auch ein unkontrolliertes Wasserlassen (Harninkontinenz) und Blut im Urin. Starker Schmerz entwikkelt sich vor allem bei voller Blase, typisch ist ein Nachlassen des Schmerzgefühls nach dem Wasserlassen. Weitere Kennzeichen sind Schmerzen des Bekkens, des unteren Bauchraumes und des Dammes, Beckendruck sowie Schmerzen beim Wasserlassen, verbunden damit, daß Urin nur tröpfchenweise abgegeben werden kann. Auch während und nach dem Geschlechtsverkehr treten häufig starke Schmerzen auf. Die Beschwerden der Patienten bedingen in nicht wenigen Fällen einen so enormen Leidensdruck, daß auch operative Verfahren bis hin zur Cystektomie in Kauf genommen werden.

Zur Diagnose der interstitiellen Cystitis ist es wichtig, daß andere Blasenerkrankungen mit ähnlichen Symptomen auszuschließen sind. In einem ersten Schritt ist zu klären, ob der Patient durch eine frühere Operation (beispielsweise im Unterbauch) schmerzbelastet ist, ob die Blasenentzündung durch eine Strahlen- oder Chemotherapie hervorgerufen wurde oder ob wiederholte, rezidivierende Infekte vorlagen oder vorliegen. In der Folge ist zu untersuchen, ob gynäkologische, neurologische, psychiatrische und/oder rheumatische Erkrankungen auszuschließen sind. Des weiteren sollten Beschwerden der Wirbelsäure und Allergien ausgeschlossen werden.

Im Rahmen der Untersuchung bzw. Diagnose der interstitiellen Cystitis kann beispielsweise in einem Labor eine Harnkultur und eine Untersuchung der zellulären Bestandteile im Urin (Harnzytologie) durchgeführt werden. Bei weiblichen Patienten sollte zum Ausschluß von sexuell übertragbaren Erkrankungen ein Vaginalabstrich erstellt werden. Die Schmerzempfindlichkeit wird über eine Tastuntersuchung der Scheide erhoben. Bei männlichen Patienten wird zum Ausschluß einer bakteriell verursachten Entzündung der Prostata eine Bakterienkultur aus dem Ejakulat angelegt. Um ein Prostatakarzinom auszuschließen, wird der Wert des prostataspezifischen Tumormarkers (PSA = prostataspezifisches Antigen) bestimmt. Über eine Ultraschalluntersuchung wird der Restharn bestimmt und ein Einwachsen der Prostata in die Blase ausgeschlossen.

Eine weitergehende Untersuchung kann auch über eine Blasenspiegelung (Cystoskopie) durchgeführt werden. Die Blasenspiegelung kann unter Narkose durchgeführt werden. Typische Zeichen für die interstitielle Cystitis, welche mittels der Cytoskopie manifestiert werden können, sind vermehrtes Einwachsen von Blutgefäßen in die Blasenschleimhaut, Flüssigkeitsansammlungen in der Schleimhaut, Aufbersten der Schleimhaut (Glomeruli), punktförmige Blutungen nach Aufdehnung der Blase unter Druck durch Wassereinspülung (Hydrodistension) sowie bei etwa 10 bis 20 % der Patienten Zeichen von Blasengeschwüren (Hunner's Ulcera).

Bei der interstitiellen Cystitis verspüren die Patienten schon bei geringen Urinmengen einen starken Harndrang; ihre Blasenkapazität ist vermindert. Zur Diagnose der interstitiellen Cystitis kann deshalb die Bestimmung des maximalen Füllvolumens und anschließend eine vergleichende Blasenkapazitätsmessung (Cystometrie) durchgeführt werden.

Untersuchungen zur interstitiellen Cystitis zeigen, daß das Blasenepithel bzw. das Urothel der Harnblase bei Vorliegen einer Cystitis defizient ist. Diese Schwächung trägt zu den klinischen Symptomen der interstitielle Cystitis wesentlich bei.

Was die Therapie der interstitiellen Cystitis anbelangt, so gibt es bis zum gegenwärtigen Zeitpunkt weder ein Heilmittel, noch eine Behandlungsmethode, die für alle Patienten wirksam ist.

So ist im Stand der Technik eine Zusammensetzung auf Basis von PentosanPolysulfat-Natrium bekannt. Man geht davon aus, daß die Wirkungsweise in der Reparatur einer dünnen oder beschädigten Blasenwand besteht. Die Erfolge sind aber nicht immer befriedigend.

Auch Antidepressiva, wie trizyklische Antidepressiva, haben sich in gewisser Weise als wirksam zur Linderung der Schmerzen und Miktionshäufigkeit bei interstitieller Cystitis erwiesen. Bei der interstitiellen Cystitis werden diese Medikamente jedoch nur wegen ihrer schmerzlindernden Eigenschaften verwendet.

Weitere orale Arzneimittel umfassen entzündungshemmende Mittel, Antispasmodika, Antihistaminika und muskelentspannende Mittel. Derartige Medikamente können die Erkrankung jedoch nur im bestimmten Maße lindern. Ein durchgreifender Therapieerfolg ist mit diesen Medikamenten in der Regel nicht möglich.

Weiterhin können Blaseninstillationen mit bestimmten Substanzen durchgeführt werden. So kann eine Blasendehnung realisiert werden, wobei die Blase unter Vollnarkose zur Dehnung mit Wasser gefüllt wird. Dies gehört zwar vorrangig zum Diagnoseverfahren für die interstitiellen Cystitis, es kann aber auch therapeutisch eingesetzt werden.

Darüber hinaus kann DMSO (Dimethylsulfoxid) als Arzneimittel direkt in die Blase gefüllt werden. Es soll entzündungshemmend wirken und somit die Schmerzen verringern. DMSO kann mit Steroiden, Heparin und/oder lokalen Anästhetika zu einem "Blasencocktail" gemischt werden. Die Nebenwirkungen sind jedoch oftmals hoch.

Weiterhin kann BCG (Bacillus Calmette-Guerin) zur Stärkung bzw. Anregung des Immunsystems instilliert werden. Diese experimentelle Behandlung befindet sich jedoch in der klinischen Untcrsuchungsphase und ist bislang noch nicht zur Behandlung von interstitieller Cystitis zugelassen.

Andere Blaseninstillationen, beispielsweise unter Verwendung von Oxychlorosen-Natrium sind größtenteils sehr schmerzhaft und erfordern eine Vollnarkose. Silbernitrat wird selten verwendet und gilt als veraltete Therapie.

Andere Behandlungsmethoden, wie eine gezielte Ernährung, wobei bestimmte Nahrungsmittel, insbesondere säurehaltige, scharfe Nahrungsmittel, vermieden werden, können die Schwere der Symptome nur geringfügig lindern. Die interstitielle Cystitis kann auch durch Rauchen, Kaffee oder Tee und alkoholische Getränke verschlimmert werden.

Selbsthilfetechniken können die Lebensqualität im geringen Umfang verbessern und die Inzidenz und Schwere von Anfällen verringern. Dazu gehören beispielsweise eine änderungen im Lebensstil, Streßreduzierung, Visualisierung, Biofeedback, Blasentraining und sportliche Betätigung. Ein dauerhafter Therapieerfolg ist mit diesen Methoden jedoch oftmals nicht möglich.

Für eine geringe Anzahl an Patienten mit schweren Symptomen, die nicht auf andere Behandlungsmethoden ansprechen, kann eine Blasenoperation in Erwägung gezogen werden. In manchen Fällen werden die Symptome jedoch auch dadurch nicht besser. Zur Behandlung der interstitiellen Cystitis wurden mehrere Arten von Operationen eingesetzt, einschließlich Cystektomie und Harnumleitung. Aufgrund der schwere des operativen Eingriffs sollten Operationen jedoch stets das letzte Mittel darstellen.

Die Therapiemöglichkeiten sind somit ebenso vielfältig wie insgesamt unbefriedigend. Zusammenfassend und in Ergänzung zu den obigen Ausführungen kommen bislang die folgenden Behandlungsmethoden für die interstitielle Cystitis in Betracht. Die Innervation beeinflussende Medikamente (Analgetika, Antispasmodika, Antihistaminika); eine cytodestruktive Therapie mit anschließender Regeneration (z. B. DMSO, BCG, Hydrodistension); eine cytoprotektive Therapie zur Wiederherstellung der Glykosaminoglykanschicht (Heparin, Pentosulfanpolysulfat). Im Rahmen der konservativen Therapie geht es um die Reduktion der Symptome mittels oral verabreichter Substanzen, wie Antispasmodika (Erfolg gering); Analgetika; Antihistaminika; Antidepressiva, vor allem Trizyklika (Amitriptylin); Zytoprotektiva, wie Pentosulfanpolysulfat (sehr lange Latenzzeit bis 2 Jahre, bis ein Erfolg meßbar wird); Immunsuppresiva, wie Azathioprin, Cyclosporin, Chloroquin; Calciumantagonisten, z. B. Nifedipin. Die Hydrodistension ist die überdehnung der Blase mittels eines intravesikal eingeführten Ballons. In der Regel wird über einen Zeitraum von drei Stunden gedehnt, die Wirkung ist jedoch gering und kurz anhaltend. Weiter gibt es verschiedene intravesikale pharmakotherapeutische Maßnahmen, insbesondere zur Wiederherstellung der Glykosaminoglykanschicht. Hierzu werden, wie zuvor erörtert, Pentosanpolysulfat oder Heparin eingesetzt; DMSO (analgetisch, antiphlogistisch, muskelrelaxierend); immunmodulierend BCG. Auch alternative Behandlungsverfahren werden durchgeführt. Dazu zählen Entspannungsübungen, Verhaltenstraining, Akupunktur, Neuromodulation und diätetische Maßnahmen.

Im Stand der Technik werden zur Behandlung von Cystitis, wie der interstitiellen Cystitis, weitere Zusammensetzungen bzw. Verfahren vorgeschlagen.

So betrifft die DE 696 29 553 T2, welche auf die europäische Patentschrift EP 0 813 417 B1 zurückgeht, eine Zusammensetzung zur Herstellung eines Arzneimittels, welches zur Behandlung der interstitiellen Blasenentzündung oder -Verletzung geeignet sein soll. Die dort beschriebene Lösung enthält Hyaluronsäure mit einem sehr hohen Molekulargewicht von mindestens 2·10⁵ Dalton, wobei ein darüber noch hinausgehendes Molekulargewicht von bis zu 1,9·10⁶ bevorzugt wird. In dieser Druckschrift liegt die Hyaluronsäure als alleinige Wirksubstanz vor und dies mit einem sehr hohen Molekulargewicht. Aufgrund des hohen Molekulargewichts der Hyaluronsäure kann aufgrund der erhöhten Viskosität der Lösung eine Verschlechterung der Applizierbarkeit bzw. Instillationsfähigkeit resultieren. Hochmolekulare Hyaluronsäure wird in diesem Dokument als alleiniger Wirkstoff eingesetzt, so daß die Wirksamkeit nicht immer optimal ist. Insbesondere ist bei der dort beschriebenen hochmolekularen Hyaluronsäure nicht immer eine optimale Interaktion mit dem Urothel gegeben.

Weiterhin betrifft die WO 2004/073584 A2 eine pharmazeutische Zusammensetzung für den Einsatz in die Blase eines Patienten, wobei die Zusammensetzung Chondroitinsulfat in hohen Mengen bzw. Konzentrationen von 250 mg bis 1.200 mg bei einem Volumen des wäßrigen Trägers von 10 ml bis 100 ml umfaßt. Auch dieses Dokument fokussiert auf die Verwendung eines einzelnen Wirkstoffs und dies in hohen Konzentrationen.

Schließlich betrifft die US 6 083 933 A Chondroitinsulfat enthaltende Zusammensetzungen, welche im Rahmen der Behandlung der interstitiellen Cystitis eingesetzt werden können, wobei in bezug auf das Chondroitinsulfat Konzentrationen von 0,1 bis 100 g/ml vorgesehen sind. Durch die Verwendung einer Monozusammensetzung, welche außer Chondroitinsulfat keine weiteren Wirkstoffe enthält, ist die Wirkung hinsichtlich der zu behandelnden Cystitiden jedoch nicht immer ausreichend.

Die US 5 880 108 A1 betrifft ein Verfahren zur Behandlung von Cystitis, wobei die Harnblase und die damit in Verbindung stehenden Strukturen mit einer Lösung in Kontakt gebracht werden sollen, Die Lösung weist Hyaluronsäure auf, deren mittleres Molekulargewicht mindestens 2·10⁵ Dalton beträgt. Die Zusammensetzung kann weiterhin bestimmte Substanzen enthalten, welche sich für die Behandlung von mit Cystitis im Zusammenhang stehenden Grunderkrankungen eignen.

Die WO 96/25168 A1 betrifft ein Vorfahren zur Behandlung von interstitieller Cystitis, wobei eine innere Blase und damit in Verbindung stehende Strukturen mit einer Lösung in Kontakt gebracht werden sollen, welche Hyaluronsäure mit einem mittleren Molekulargewicht von nicht weniger als 2·10⁵ Dalton enthält.

Weiterhin betrifft die US 2006/0234978 A1 eine Zusammensetzung, welche sich zur Behandlung bzw. Prävention von interstitieller Cystitis oder von hiermit in Beziehung stehenden Erkrankungen des Urogenitaltraktes eignen soll, wobei die Zusammensetzung in therapeutischen Mengen Chondroitinsulfat in Kombination mit Hyaluronsäure aufweisen soll.

Die WO 03/034993 A2 offenbart eine Zusammensetzung, welche in verschiedenen Applikationsformen, wie in Form eines Gels, eines Sprays, eines Puders, eines Schaums oder in Form einer injektionsfähigen Flüssigkeit bzw. einer oral aufnehmbaren Flüssigkeit, vorliegen kann. Die Zusammensetzung soll zudem ein polysulfatiertes Glykosaminoglykan, eine Hyaluronsäure und ein Glukosaminsalz aufweisen, wobei es sich bei dem polysulfatierten Glykosaminoglykan um Chondroitinsulfat handeln kann.

Schließlich betrifft die EP 1 124 546 B1 die Verwendung einer Zusammensetzung zur Herstellung eines Arzneimittels zur Verwendung bei der Prävention von Blasenentzündung, hervorgerufen durch Radiotherapie des Blasenbereichs, wobei das Arzneimittel vor einer Radiotherapiebehandlung des Blasenbereichs eingesetzt werden soll. Die diesbezügliche Zusammensetzung soll Hyaluronsäure mit einem durchschnittlichen Molekulargewicht von nicht weniger als 2·10⁵ Dalton und einen pharmazeutisch annehmbaren Trägerstoff umfassen.

Somit liegt der vorliegenden Erfindung das Problem zugrunde, eine Zubereitung, insbesondere pharmazeutische Zubereitung bereitzustellen, welche sich zur Behandlung von entzündlichen Erkrankungen des Urogenitaltraktes, vorzugsweise von Cystitis, besonders bevorzugt von interstitieller Cystitis, eignet und welche die zuvor geschilderten Nachteile des Standes der Technik zumindest weitgehend vermeidet oder aber wenigstens abschwächt. Dabei soll insbesondere eine bessere Wirksamkeit sowie eine erleichterte Applikation bzw. Instillation der Zubereitung gewährleistet sein.

Zur Lösung der zuvor geschilderten Aufgabe schlägt die vorliegende Erfindung eine Zubereitung nach Anspruch 1 vor; weitere vorteilhafte Ausgestaltungen sind Gegenstand der diesbezüglichen abhängigen Ansprüche. Weiterer Gegenstand der vorliegenden Erfindung ist ein Behältnis nach Anspruch 13, welches die erfindungsgemäße Zubereitung enthält. Wiederum weiterer Gegenstand der vorliegenden Erfindung ist eine Applikationsvorrichtung zur Instillation nach Anspruch 14, welches die erfindungsgemäße Zubereitung enthält. Schließlich ist weiterer Gegenstand der vorliegenden Erfindung die Verwendung der erfindungsgemäßen Zubereitung nach Anspruch 14.

Gegenstand der vorliegenden Erfindung - gemäß einem ersten Aspekt der vorliegenden Erfindung - ist somit eine Zubereitung, welche sich insbesondere zur Behandlung von entzündlichen Erkrankungen des Urogenitaltraktes, vorzugsweise von Cystitis, eignet und in Kombination und jeweils in pharmazeutisch wirksamen Mengen
- Hyaluronsäure und/oder deren Salze mit einer mittleren Molmasse (Molekulargewicht) von weniger als 200 kDa und
- mindestens ein Chondroitinsulfat
enthält.

Der Begriff "pharmazeutische Zubereitung" oder dergleichen, wie er im Rahmen der vorliegenden Erfindung verwendet wird, ist sehr umfassend zu verstehen und bezeichnet nicht nur pharmazeutische Präparate bzw. Pharmazeutika als solche, sondern auch sogenannte Medizinprodukte, homöopathische Mittel oder dergleichen.

Bei allen nachstehend genannten relativen bzw. prozentualen gewichtsbezogenen Mengenangaben ist zu beachten, daß diese im Rahmen der erfindungsgemäßen Zubereitung bzw. Kombination vom Fachmann derart auszuwählen sind, daß sie sich in der Summe unter Einbezug der anderen Komponenten bzw. Inhaltsstoffe bzw. Bestandteile, insbesondere wie nachfolgend definiert, stets um 100 Gew.-% ergänzen. Dies versteht sich für den Fachmann aber von selbst.

Die vorliegende Erfindung zeichnet sich durch die Verwendung einer speziellen Hyaluronsäure mit relativ geringer mittlerer Molmasse aus. Denn was die mittlere Molmasse - im Rahmen der vorliegenden Erfindung synonym auch als Molekulargewicht bezeichnet - der erfindungsgemäß verwendeten Hyaluronsäure bzw. deren Salze betrifft, so kann diese im Rahmen der vorliegenden Erfindung in einem Bereich von 50 bis 195 kDa (Kilodalton), insbesondere 100 bis 195 kDa, vorzugsweise 150 bis 190 kDa, bevorzugt 175 bis 190 kDa, liegen. So kann erfindungsgemäß beispielsweise eine Hyaluronsäure bzw. deren Salze mit einem Molekulargewicht von etwa 180 kDa verwendet werden.

Denn die Anmelderin hat in völlig überraschender Weise herausgefunden, daß durch den Einsatz einer speziellen Hyaluronsäure bzw. deren Salzen der vorgenannten Art hinsichtlich der Behandlung von Cystitis besonders gute Ergebnisse erzielt werden. Diesbezüglich kommt auch dem Molekulargewicht eine entscheidende Rolle zu. Ohne sich auf eine Theorie festlegen zu wollen, kann dies damit begründet werden, daß die Hyaluronsäure bzw. deren Salze mit geringerem Molekulargewicht gewissermaßen besser in die geschädigten Urothel- bzw. Zellschichtstrukturen der Harnblase eindringen bzw. besser mit diesen interagieren und somit sozusagen die Hamblaseninnenwandung besser "auskleiden" bzw. abdecken können, so daß hierdurch ein optimaler Schutzeffekt einhergehend mit einem signifikant verbesserten Krankheitsverlauf erzielt wird. Darüber hinaus ist durch die Verwendung von Hyaluronsäure bzw, deren Salzen mit einem Molekulargewicht von weniger als 200 kDa einerseits die Herstellung der erfindungsgemäßen Zubereitung verbessert, da eine stabilere Lösung bzw. Suspension realisiert werden kann, so daß auch deren Lagerfähigkeit verbessert ist. Andererseits kann die erfindungsgemäße Zubereitung beispielsweise aufgrund der verbesserten Viskosität einfacher instilliert bzw. vorzugsweise topisch appliziert werden.

Der volumenbezogene Wirkstoffgehalt an Hyaluronsäure bzw. deren Salzen beträgt 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, bezogen auf das Volumen der vorzugsweise in flüssiger Form vorliegenden Zubereitung nach der Erfindung. Dies bedeutet, daß bei einem erfindungsgemäß bevorzugt applizierten Volumen der erfindungsgemäßen Zubereitung von 50 ml 10 bis 100 mg, insbesondere 10 bis 80 mg, bevorzugt 20 bis 60 mg, besonders bevorzugt 25 bis 50 mg, besonders bevorzugt 35 bis 45 mg Hyaluronsäure und/oder deren Salze in der Zubereitung nach der Erfindung mit einem Volumen von 50 ml enthalten sind. Gemäß einer erfindungsgemäß ganz besonders bevorzugten Ausführungsform enthält die Zubereitung nach der Erfindung bei einem Volumen von 50 ml etwa 40 mg Hyaluronsäure bzw. deren Salze.

Was die erfindungsgemäße Zubereitung weiterhin anbelangt, so kann diese die Hyaluronsäure bzw. deren Salze in relativen, gewichtsbezogenen Mengen von 0,02 bis 0,2 Gew.-%, insbesondere 0,03 bis 0,16 Gew.-%, vorzugsweise 0,04 bis 0,12 Cew.-%, bevorzugt 0,05 bis 0,10 Gew.-%, bezogen auf das Gesamtgewicht der Zubereitung vorhanden sein. Dennoch kann es einzelfallbedingt oder anwendungsbezogen erforderlich sein, von den vorgenannten Mengen abzuweichen, ohne daß der Rahmen der vorliegenden Erfindung verlassen ist. Somit kann die Menge bzw. an Hyaluronsäure bzw. deren Salze in weiten Bereichen variieren.

Die erfindungsgemäße Zubereitung zeichnet sich dadurch aus, daß die verwendete Hyaluronsäure bzw. deren Salze bevorzugt nichttierischen Ursprungs ist. So kann die Hyaluronsäure bzw. deren Salze beispielsweise bakteriellen Ursprungs sein. Diesbezüglich kann die Hyaluronsäure und/oder deren Salze z. B. fermentativ von Bakterien der Gattung *Streptococcus,* insbesondere *Streptococcus lancefields,* bevorzugt *Streptococcus lancefields* Stamm A, gewonnen sein. Die diesbezügliche Isolierung der Wirksubstanzen ist dem Fachmann als solche geläufig. Weiterhin kann die erfindungsgemäß verwendete Hyaluronsäure aber auch pflanzlichen Ursprungs sein.

Die Anmelderin hat nicht zuletzt aufgrund ihrer intensiven Forschungstätigkeit in überraschender Weise herausgefunden, daß durch die Verwendung einer nichttierischen Hyaluronsäure die pharmazeutische Wirksamkeit der erfindungsgemäßen Zubereitung in bezug auf die Behandlung von Cystitiden, insbesondere der interstitiellen Cystitis, signifikant verbessert ist. Ohne sich diesbezüglich auf eine spezifische Theorie festlegen zu wollen, kann dies damit begründet werden, daß es sich bei der nichttierischen, insbesondere bakteriell gewonnenen Hyaluronsäure um ein besonders reines Produkt mit definierten chemischen und physikalischen Eigenschaften handelt, welches hochwirksam ist. Zudern ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. Somit weist Hyaluronsäure nichttierischen Ursprungs eine besonders hohe Reinheit und Homogenität auf.

Gemäß einer erfindungsgemäß bevorzugten Ausführungsform wird in bezug auf die erste Komponente der binären Zubereitung nach der Erfindung die Hyaluronsäure bzw. deren Salze als ein Alkalihyaluronat eingesetzt, wobei es sich diesbezüglich vorzugsweise um Natriumhyaluronat handelt.

Die erste Komponente der erfindungsgemäßen Zubereitung kann vorzugsweise auf einer sterilen und hochgereinigten Lösung oder Suspension des Natriumsalzes der Hyaluronsäure basieren.

Für weitergehende Einzelheiten zum Begriff der Hyaluronsäure bzw. deren Salzen kann auf Römpp Chemielexikon, 10. Auflage, Band 3, 1997, Georg Thieme Verlag Stuttgart/New York, Seite 1820, Stichwort: "Hyaluronsäure", sowie die dort referierte Literatur verwiesen werden, wobei der gesamte Offenbarungsgehalt der vorgenannten Literatur hiermit durch Bezugnahme eingeschlossen ist.

Was das weitere von der Hyaluronsäure bzw. deren Salzen verschiedene Glykosaminoglykan anbelangt, so handelt es sich hierbei gemäß einer erfmdungsgemäß besonder bevorzugten Ausführungsform um Chondroitinsulfat. Diesbezüglich kann das erfindungsgemäß verwendete Chondroitinsulfat in bevorzugter Weise marinen Ursprungs sein. In nichtbeschränkender Weise kann das Chondroitinsulfat marinen Ursprungs aus Knorpelfischen, beispielsweise aus Haien, gewonnen sein.

Die Anmelderin hat völlig überraschend herausgefunden, daß ein derartiges Chondroitinsulfat marinen Ursprungs insbesondere bei der Behandlung von Cystitis, vorzugsweise der interstitiellen Cystitis, zu besonders guten Ergebnissen führt, insbesondere wenn dies in Kombination mit der Hyaluronsäure der vorgenannten Art eingesetzt wird. Ohne sich diesbezüglich auf eine Theorie festlegen zu wollen, eignet sich dieses spezielle Chondroitinsulfat aufgrund seiner molekularen Struktur, wie monomeren Zusammensetzung, in besonderer Weise, um - insbesondere in Verbindung mit der Hyaluronsäure nichttierisehen Ursprungs -, die zuvor beschriebene Glykosaminoglykanschicht des Urothels zu regenerieren bzw. gewissermaßen aufzufüllen, um die Permeabilität dieser Schicht zu erniedrigen und um auf diese Weise die Schutzfunktion der Glykosaminoglykanschicht bzw. der Schleimschicht des Urothels (Mucinlayer) zu erhöhen, so daß es zu einer deutlichen Linderung bzw, Ausheilung der Krankheitssymptome kommt. Bestandteile des erfindungsgemäß verwendeten Chondroitinsulfats marinen Ursprungs können - ohne sich hierauf beschränken zu wollen - beispielsweise Chondroitin-4-sulfat und/oder Chondroitin-6-sulfat sein.

Die Anmelderin hat überraschenderweise herausgefunden, daß besonders gute Ergebnisse dann erhalten werden, wenn als weiteres, von der Hyaluronsäure und/oder deren Salzen verschiedenes Glykosaminoglykan ein Chondroitinsulfat eingesetzt wird, dessen mittlere Molmasse (mittleres Molekulargewicht) 10 bis 70 kDa, insbesondere 20 bis 60 kDa, vorzugsweise 25 bis 50 kDa, bevorzugt 30 bis 40 kDa, beträgt.

Erfindungsgemäß kann das Chondroitinsulfat ausgewählt sein aus der Gruppe von Chondroitin-4-sulfat (Chondroitinsulfat A), Chondroitin-6-sulfat (Chondroitinsulfat B) und Dermatansulfat (β-Heparin bzw. Chondroitinsulfat C) sowie deren Mischungen, vorzugsweise Chondroitin-4-sulfat und/oder Chondroitin-6-sulfat; dabei werden die vorgenannten Chondroitinsulfate erfindungsgemäß bevorzugt in Form ihrer Alkalisalze, vorzugsweise Natriumsalze eingesetzt, da dies im Rahmen der vorliegenden Erfindung die besten Ergebnisse liefert.

In erfindungsgemäß besonders bevorzugter Weise wird als weiteres, von der Hyaluronsäure bzw. deren Salzen verschiedenes Glykosaminoglykan eine Chondroitinsulfatmischung aus mindestens zwei verschiedenen Chondroitinsulfaten eingesetzt, vorzugsweise eine Mischung aus Chondroitin-4-sulfat und Chondroitin-6-sulfat, insbesondere in Form von deren Alkalisalzen, vorzugsweise Natriumsalzen. Erfindungsgemäß besonders bevorzugt ist eine Mischung aus Chondroitin-6-sulfat und Chondroitin-4-sulfat mit einem Mengenverhältnis (Gewichtsverhältnis) von Chondroitin-6-sulfat zu Chondroitin-4-sulfat von 60 - 95%: 40 - 5 %, insbesondere 75 - 95 % : 25 - 5 %, vorzugsweise 80 - 90%: 20 - 10 %, da dies im Rahmen der erfindungsgemäßen Versuche bzw. Studien die besten Therapieergebnisse liefert.

Für weitergehende Einzelheiten zum Begriff der Condroitinsulfaten kann auf Römpp Chemielexikon, 10. Auflage, Band 1, 1996, Georg Thieme Verlag Stuttgart/New York, Seite 736, Stichwort: "Chondroitinsulfate", sowie die dort referierte Literatur verwiesen werden, wobei der gesamte Offenbarungsgehalt der vorgenannten Literatur hiermit durch Bezugnahme eingeschlossen ist.

Der volumenbezogene Wirkstoffgehalt des weiteren, von der Hyaluronsäure bzw. deren Salzen verschiedenen Glykosaminoglykans, vorzugsweise Chondroitinsulfat, kann 0,2 bis 2,0 mg/ml, insbesondere 0,3 bis 1,6 mg/ml, vorzugsweise 0,4 bis 1,2 mg/ml, bevorzugt 0,1 bis 1,0 mg/ml, besonders bevorzugt 0,7 bis 0,9 mg/ml, betragen, bezogen auf das Volumen der Zubereitung. Somit kann - bezogen auf die vorzugsweise in flüssiger Form vorliegende Zubereitung - bei einem Volumen der erfindungsgemäßen Zubereitung von 50 ml das weitere, von der Hyaluronsäure bzw. deren Salzen verschiedene Glykosaminoglykan, vorzugsweise Chondroitinsulfat, in Mengen von 1 bis 100 mg, insbesondere 15 bis 80 mg, vorzugsweise 20 bis 60 mg, bevorzugt 25 bis 50 mg, besonders bevorzugt 35 bis 45 mg, vorliegen. Gemäß einer erfindungsgemäß besonders bevorzugten Ausführungsform beträgt die Menge an dem weiteren Glykosaminoglykan in der Zubereitung etwa 40 mg bei einem Volumen der erfindungsgemäßen Zubereitung von 50 ml.

Was die relative gewichtsbezogene Menge an dem weiteren, von der Hyaluronsäure bzw. deren Salzen verschiedenen Glykosaminoglykans, vorzugsweise Chondroitinsulfat, anbelangt, so kann diese erfindungsgemäß in einem Bereich von 0,02 bis 0,20 Gew.-%, insbesondere 0,03 bis 0,16 Gew.-%, vorzugsweise 0.04 bis 0,12 Gew.-%, bevorzugt 0,05 bis 0,10 Gew.-%, bezogen auf das Gesamtgewicht der Zubereitung liegen.

Im Rahmen der vorliegenden Erfindung ist es darüber hinaus gelungen, die Wirksamkeit der erfindungsgermäßen Zubereitung in bezug auf die Behandlung von Cystitis, insbesondere der interstitiellen Cystitis, durch eine gezielte Abstimmung des Mengenverhältnisses zwischen Hyaluronsäure bzw. deren Salzen einerseits und dem von der Hyaluronsäure bzw. deren Salzen verschiedenen Glykosaminoglykans, vorzugsweise Chondroitinsulfat, andererseits zu erhöhen. So kann es erfindungsgemäß vorgesehen sein, daß das Mengenverhältnis von Hyaluronsäure bzw. deren Salzen und dem weiteren, von der Hyaluronsäure bzw. deren Salzen verschiedenen Glykosaminoglykan, vorzugsweise Chondroitinsulfat, im Bereich von 0,25: 2 bis 2; 0,25, insbesondere 0,5 : 1,5 bis 1,5 : 0,5, vorzugsweise 0,75: 1,25 bis 1,25 : 0,75, liegt. Gemäß einer erfindungsgemäß besonders bevorzugten Ausführungsform beträgt das vorgenannte Verhältnis etwa 1:1, wobei diesbezüglich auch hiervon geringfügig abweichende Mengenverhältnisse mitumfaßt sind. Denn der Anmelderin ist es gelungen, durch eine gezielte Anpassung und Abstimmung der jeweiligen Wirksubstanzen der binären erfindungsgemäßen Zubereitung in bezug auf die Glykosaminoglykanschicht des Blasenurothels zu einer besonders guten Wirksamkeit hinsichtlich der Behandlung der vorgenannten Erkrankungen zu gelangen, da - ohne sich auf eine Theorie festlegen zu wollen - insbesondere bei einem Mengenverhältnis von etwa 1: 1 eine besonders gute Integration der Wirksubstanzen in die Glykosaminoglykanschicht des Blasenurothels bzw. eine gute Regeneration bzw, ein guter Aufbau der Glykosaminoglykanschicht des Blasenurothels vorliegt. Auf diese Weise wird - wie zuvor angeführt - die Permeabilität des Urothels in signifikanter Weise erniedrigt, was zu einer deutlichen Linderung der mit der interstitiellen Cystitis einhergehenden Symptome verbunden ist, insbesondere da Reizstoffe nicht mehr so tief in das Urothel bzw. in darunter liegende Schichten eindringen können.

In Ergänzung zu den vorgenannten Wirksubstanzen kann die erfindungsgemäße Zubereitung weiterhin mindestens einen Elektrolyten enthalten, welcher insbesondere in Mengen von 0,001 bis 1 Gew.-%, insbesondere 0,005 bis 0,03 Gew.-%, vorzugsweise 0,007 bis 0,02 Gew.-%, bevorzugt 0,01 bis 0,017 Gew.-% vorliegt, bezogen auf das Gesamtgewicht der Zubereitung. Hierbei ist insbesondere darauf abzustellen, daß mögliche osmotische Effekte so gering wie möglich gehalten werden, was die Verträglichkeit der erfindungsgemäßen Zubereitung weiter erhöht.

Bei dem erfindungsgemäß fakultativ vorgesehenen Elektrolyten kann es sich um einen Elektrolyten handeln, welcher aus organischen bzw. anorganischen Alkali- oder Erdalkalisalzen sowie deren Mischungen ausgewählt ist. Vorzugsweise handelt es sich bei dem Elektrolyten um ein Alkali- oder Erdalkalisalz. Gemäß einer erfindungsgemäß besonders bevorzugten Ausführungsform handelt es sich bei dem Alkali- bzw. Erdalkalisalz um ein Chlorid und vorzugsweise um Natriumchlorid. Diesbezüglich ist es erfindungsgemäß besonders bevorzugt, wenn der Elektrolyt, vorzugsweise Natriumchlorid, in der vorzugsweise flüssigen erfindungsgemäßen Zubereitung in einer Menge von etwa 6,9 mg bei einem Volumen von 50 ml vorhanden ist. Einzelfallbedingt oder anwendungsbezogen kann es dennoch erforderlich sein, von den vorgenannten Mengen abzuweichen, ohne daß der Rahmen der vorliegenden Erfindung verlassen ist.

Die erfindungsgemäße Zusammensetzung ist nicht auf die vorgenannten Wirkstoffe bzw. Substanzen beschränkt. So kann es erfindungsgemäß vorgesehen sein, daß die Zubereitung nach der Erfindung weitere Substanzen enthält:

So kann die erfindungsgemäße Zubereitung beispielsweise außerdem Dexpanthenol bzw. dessen Derivate, insbesondere Ester, enthalten. Die diesbezüglichen Mengen können im Bereich von 0,0001 bis 5 Gew.-%, vorzugsweise 0,001 bis 4 Gew.-%, bevorzugt 0,01 bis 3 Gew.-%, besonders bevorzugt 0,1 bis 2 Gew.-%, liegen, bezogen auf das Gesamtgewicht der Zubereitung. Bei Dexpanthenol handelt es sich um den internationalen Freinamen für D-(+)-2,4-Dihydroxy-N-(3-hydroxypropyl)-3,3-dimethylbutyramid. Hierbei handelt es sich um eine wundheilend wirkende Substanz, welche die Wirkung der erfindungsgemäßen Zubereitung unterstützen bzw. steigern kann, beispielsweise durch ein verbessertes Ausheilen von Läsionen der Urothelschicht, welche im Rahmen einer Cystitis häufig zu beobachten ist.

Weiterhin kann die erfindungsgemäße Zubereitung außerdern zusätzlich Ectoin bzw. Ectoinderivate, wie Hydroxyectoin, enthalten. Die diesbezüglichen Mengen können im Bereich von 0,001 bis 5 Gew.-%, vorzugsweise 0,001 bis 4 Gew.-%, bevorzugt 0,1 bis 3 Gew.-%, besonders bevorzugt 0,1 bis 2 Gew.%, variieren, bezogen auf das Gesamtgewicht der Zubereitung. Bei Ectoin bzw. Ectoinderivaten, wie Hydroxyectoin, handelt es sich um Wirksubstanzen zur Behandlung von Hauterkrankungen. Derartige Substanzen können die Wirksamkeit der erfindungsgemäßen Zubereitung weiter unterstützen, insbesondere hinsichtlich ihrer positiven Wirkung in bezug auf das Urothel. Für weitere diesbezügliche Ausführungen in bezug auf Ectoin kann verwiesen werden auf die europäische Patentschrift EP 0 887 418 B1, deren gesamter diesbezüglicher Offenbarungsgehalt hiermit durch Bezugnahme eingeschlossen ist.

Weiterhin kann es vorgesehen sein, der erfindungsgemäßen Zubereitung - als alleinige zusätzliche Wirksubstanz oder in Ergänzung zu den vorgenannten Wirkstoffen - außerdem mindestens ein Lokalanästhetikum zuzugeben. Der Fachmann ist jederzeit in der Lage, Lokalanästhetika auszuwählen, die in bezug auf die vorliegende Erfindung besonders geeignet sind. Die diesbezüglichen Mengen eines Lokalanästhetikums können erfindungsgemäß im Bereich von 0,0001 bis 5 Gew.-%, vorzugsweise 0.001 bis 4 Gew.-%, bevorzugt 0,01 bis 3 Gew.-%, besonders bevorzugt 0,1 bis 2 Gew.-%, liegen, bezogen auf das Gesamtgewicht der Zubereitung. Die Zugabe eines Lokalanästhetikums kann die Wirkung der erfindungsgemäßen Zubereitung dahingehend unterstützen bzw. verbessern, daß im Zusammenhang mit der Instillation bzw. Applikation der erfindungsgemäßen Zubereitung auch eine gewisse Schmerzstillung eintritt. Dies ist besonders vorteilhaft und führt zu einem erhöhten Wohlbefinden von mit der erfindungsgemäßen Zubereitung behandelten, unter einer Cystitis leidenden Personen, insbesondere da die zu behandelnden Erkrankungen oftmals von mitunter starken schmerzsymptomen begleitet sind.

Schließlich kann die erfindungsgemäße Zubereitung außerdem auch Mineralstoffe und/oder Vitamine enthalten. Insbesondere können Zinkverbindungen, vorzugsweise organische Zinkverbindungen, bevorzugt Zinkgluconat, eingesetzt werden. Die diesbezüglichen Mengen können im Bereich von 0,0001 bis 5 Gew.-%, vorzugsweise 0,001 bis 4 Gew.-%, bevorzugt 0,01 bis 3 Gew.-%, besonders bevorzugt 0,1 bis 2 Gew.%, liegen, bezogen auf das Gesamtgewicht der Zubereitung Zinkverbindungen der vorgenannten Art weisen eine entzündungshemmende Wirkung auf.

Die erfindungsgemäße Zubereitung kann insbesondere eine Osmolarität von 50 bis 1000 mOsmol/l, insbesondere 100 bis 800 mOsmol/l, vorzugsweise 150 bis 600 mOsmol/l, bevorzugt 200 bis 400 mOsmol/l, aufweisen. Darüber hinaus kann die Zubereitung nach der Erfindung insbesondere einen pH-Wert von 6 bis 8, insbesondere 6,5 bis 7,8, vorzugsweise 7 bis 7,5, aufweisen. Aufgrund der vorgenannten Eigenschaften ist die erfindungsgemäße Zubereitung besonders gut verträglich und führt zu keinen nennenswerten Nebenwirkungen. In diesem Zusammenhang ist zudem festzustellen, daß Hyaluronsäure an sich zumindest im wesentlichen keine potentiell immunogenen Strukturen enthält.

Was die Zubereitung anbelarigt, so kann diese bei einer Temperatur von 20 °C eine dynamische Viskosität von 5.000 bis 12.000 mPas, insbesondere 6.000 bis 10.000 mPas, vorzugsweise 7.500 bis 9.500 mPas, bevorzugt 8.000 bis 9.000 mPas, aufweisen. Aufgrund der erfindungsgemäß vorgesehenen Viskosität ist eine besonders einfache und weniger schmerzhafte Instillation der Zubereitung in die Harnblase gewährleistet. Weiterhin liegt aufgrund der erfindungsgemäß vorgesehenen Viskosität eine besonders gute Interaktion der Zubereitung nach der Erfindung mit dem Urothel vor, was die Wirksamkeit positiv beeinflußt.

Neben den vorgenannten Wirk- und/oder Inhaltsstoffen kann die erfindungsgemäße Zubereitung außerdem übliche pharmazeutische Zusatz und/oder Hilfsstoffe enthalten. Diese können insbesondere ausgewählt sein aus der Gruppe von Stabilisierungs-, Färbe-, Puffer-, Kriech-, Streck-, Binde-, Netz- und/oder Konsetvierungsstoffen und deren Kombinationen. Der Fachmann ist jederzeit in der Lage die diesbezüglich zu wählenden Mengen in bezug auf die erfindungsgemäße Zubereitung anzupassen.

Die erfindungsgemäße Zubereitung liegt vorzugsweise in flüssiger Form vor, beispielsweise in Form einer vorzugsweise wäßrigen Lösung bzw. in Form einer vorzugsweise wäßrigen Suspension, wobei Wasser als Lösernittel erfindungsgemäß bevorzugt ist. Aufgrund des Vorliegens der Zubereitung in flüssiger Form eignet sich die erfindungsgemäße Zubereitung zur vorzugsweise topischen Applikation bzw. zur Instillation insbesondere mittels hierfür geeigneter Applikations- bzw. Instillationsvorrichtungen in den Urogenitalbercich, insbesondere in die Harnblase. Dabei ist es selbstverständlich, daß die erfindungsgemäße Zubereitung steril ist, um während der Anwendung bakterielle Infektionen zu vermeiden.

Gemäß einer erfindungsgemäß besonders bevorzugten Ausführungsform ist die Zubereitung nach der vorliegenden Erfindung, welche vorzugsweise in Form einer wäßrigen Lösung und/oder einer wäßrigen Suspension vorliegt, durch die folgende Rezeptur gekennzeichnet, wobei alle nachstehend genannten Mengenangaben jeweils auf die erfindungsgemäße Zubereitung bezogen sind und die Summe aller Bestandteile vom Fachmann selbstverständlich derart zu kombinieren sind, daß sie in der Summe zu 100 Gew.-%, wobei die Rezeptur in Kombination enthält:
- Hyaluronsäure und/oder deren Salze, insbesondere nichttierischen Ursprungs, vorzugsweise Natriumhyaluronat, bevorzugt Natriumhyaluronat nichttierischen Ursprungs, insbesondere mit einer mittleren Molmasse (Molekulargewicht) von weniger als 200 kDa, insbesondere mit einer Molmasse im Bereich von 50 bis 195 kDa, insbesondere 100 bis 195 kDa, vorzugsweise 150 bis 190 kDa, bevorzugt 175 bis 190 kDa und/oder insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, bezogen auf das Volumen der Zubereitung,
- mindestens ein weiteres, hiervon verschiedenes Glykosaminoglykan, vorzugsweise mindestens ein Chondroitinsulfat, insbesondere marinen Ursprungs, insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1.0 mg/ml, besonders bevorzugt 0,7 bis 0,9 mg/ml, und
- gegebenenfalls mindestens einen Elektrolyten, vorzugsweise Natriumchlorid, insbesondere mit einem Gehalt von 0,05 bis 0,3 mg/ml, insbesondere 0,07 bis 0,2 mg/ml, vorzugsweise 0,1 bis 0,17 mg/ml,
insbesondere wobei das Mengenverhältnis (Gewichtsverhältnis) von Hyaluronsäure und/oder deren Salzen, vorzugsweise Natriumhyaluronat, einerseits zu dem weiteren, von der Hyaluronsäure und/oder deren Salzen verschiedenen Glykosaminoglykan, vorzugsweise Chondroitinsulfat, andererseits in der Zubereitung im Bereich von 0,25 : 2 bis 2 : 0,25, insbesondere 0,5 : 1,5 bis 1,5 : 0,5, vorzugsweise 0,75 : 1,25 bis 1,25 : 0,75, besonders bevorzugt etwa 1 : 1, liegt.

Gemäß einer bevorzugten Ausführungsform der vorliegenden Erfindung enthalten 50 ml der erfindungsgemäßen Zubereitung:

| | |
|---|---|
| Natricunhyaluronat | 40 mg |
| Chondroitinsulfat | 40 mg |
| Natriumchlorid | 6,9 mg |

in vorzugsweise wäßriger Lösung.

Wie zuvor beschrieben, eignet sich die erfindungsgemäße Zubereitung zur prophylaktisch und/oder therapeutischen Behandlung von Cystitis vorzugsweise nichtbakteriellen Ursprungs, insbesondere akuter oder chronischer Cystitis, wobei hier insbesondere die zuvor beschriebene interstitielle Cystitis, radiogene Cystitis und chronische bzw. chronifizierte bakterielle Cystitis zu nennen ist. Erfindungsgemäß bevorzugt eignet sich die Zubereitung nach der Erfindung vorzugsweise zur Behandlung von interstitieller Cystitis. Was die Zubereitung nach der Erfindung weiterhin anbelangt, so ist diese vorzugsweise dosierfertig in Behältnisse eingebracht, wobei diesbezüglich insbesondere Volumengrößen von 30 bis 70 ml, insbesondere 40 bis 60 ml, bevorzugt etwa 50 ml, je Behältnis bzw. Applilcationseinheit bzw. Dosiereinheit vorgesehen sein können.

Die vorliegende Erfindung betrifft - gemäß einem **weiteren** Aspekt der vorliegenden Erfindung - gleichermaßen ein Behältnis, welches die erfindungsgemäße Zubereitung enthält. Das erfindungsgemäße Behältnis, welches die Zubereitung nach der Erfindung enthält, kann insbesondere in Form einer Durchstechflasche, vorzugsweise mit sterilem Verschluß, vorliegen.

Gemäß einem wieder **weiteren** Aspekt betrifft die vorliegende Erfindung gleichermaßen eine Applikationsvorrichtung zur Instillation bzw. zur topischen Applikation der erfindungsgemäßen Zubereitung in den Urogenitalbereich, insbesondere in die Harnblase. Die erfindungsgemäße Applikationsvorrichtung enthält die erfindungsgemäße Zubereitung. Bei der erfindungsgemäßen Applikationsvorrichtung kann es sich insbesondere um eine Applikationsvorrichtung zur einmaligen Anwendung handeln, welche zum Beispiel und in nichtbeschränkender Weise ein Aufnahme- bzw. Vorratsbehältnis zur Aufnahme der erfindungsgemäßen Zubereitung sowie eine Applikationseinrichtung, beispielsweise einen Instillationsschlauch bzw. einen Katheter oder dergleichen, enthält. Bei der Applikationsvorrichtung kann es sich beispielsweise um eine vorzugsweise sterile Spritze handeln, welche die erfindungsgemäße Zubereitung enthalten kann. Die erfindungsgemäße Spritze kann mit einer Applikationseinrichtung, beispielsweise mit einem Instillationsschlauch bzw. einem Katheter oder dergleichen, ausgestattet sein bzw. fest mit dieser verbunden sein, insbesondere um eine Instillation bzw. Applikation der Zubereitung nach der Erfindung in die Harnblase zu ermöglichen. Die erfindungsgemäße Spritze kann vorzugsweise nach Art einer Einmaldosiervorrichtung bzw. Einmalapplikationsvorrichtung konzipiert sein.

Weiterer Gegenstand der vorliegenden Erfindung - gemäß einem noch weiteren Aspekt der vorliegenden Erfindung - ist die Verwendung der Zubereitung nach der Erfindung zur prophylaktischen oder therapeutischen Behandlung von Erkrankungen des Urogenitattraktes, insbesondere von entzündlichen Erkrankungen der Harnblase. Hierbei handelt es sich vorzugsweise um Cystitis, bevorzugt um interstitielle Cystitis, radiogene Cystitis und/oder chronifizierte bakterielle Cystitis. Eine erfindungsgemäß besonders bevorzugte Verwendung der Zubereitung nach der Erfindung ist die prophylaktische oder therapeutische Behandlung von interstitieller Cystitis. Erfindungsgemäß eignet sich die Zubereitung nach der Erfindung somit insbesondere zur Behandlung von Cystitis nichtbakteriellen Ursprungs.

Denn die erfindungsgemäße Kombination, welche Hyaluronsäure bzw. deren Salze, vorzugsweise mit einer Molmasse von weniger als 200 kDa, und das hiervon verschiedene Glykosaminoglykan, vorzugsweise Chondroitinsulfat, enthält, führt zu einer besonders guten Wirksamkeit hinsichtlich der vorgenannten Erkrankungen. Eine mögliche Erklärung für die hervorragende Wirkung der erfindungsgemäßen Zubereitung kann- ohne sich hierauf festlegen zu wollen - darin gesehen werden, daß die Wirksubstanzen der erfindungsgemäßen Zubereitung- Hyaluronsäure bzw. deren Salze einerseits und das weitere, hiervon verschiedene Glykoswninoglykan, insbesondere Chondroitinsulfat, andererseits - in besonders effektiver Weise insbesondere mit dem Urothel der Harnblase wechselwirken, wobei diesbezüglich eine An- bzw. Einlagerung der Wirksubstanzen in diese Schicht vorliegt. Hierdurch wird die insbesondere durch die Erkrankung geschädigte Glykosaminoglykanschicht (Mucinlayer) des Urothels regeneriert bzw, "repariert". Die mit dieser Wirkweise verbundene Verringerung der Permeabilität - was gewissermaßen einem "Abdichteffekt" gegenüber dem in der Blase vorhandenen Harn bzw. Urin gleichkommt - führt zu einer deutlichen Linderung der krankheitsbedingten Symptome. Mit der erfindungsgemäßen Zubereitung behandelte Patienten wiesen bereits nach wenigen Behandlungen einen signifikant verbesserten Gesundheitsstatus auf, einhergehend mit einer deutlichen Verringerung der Schmerzsymptomatik. Die erfindungsgemäße Zubereitung kann somit gewissermaßen zumindest auch zum vorübergehenden Ersatz einer defekten Glykosaminoglykanschicht des Urothels dienen.

In diesem Zusammenhang kann die Wirkweise der Zubereitung nach der Erfindung somit - ohne sich hierauf festlegen zu wollen - weniger auf einen direkten pharmakologischen Effekt auf das Gewebe der Harnblase nach Einbringung der erfindungsgemäßen Zubereitung zurückgeführt werden. Vielmehr beruht die Wirkung der erfindungsgemäßen Zubereitung in erster Linie auf einer im wesentlichen physikalischen Wechselwirkung, bei welcher Hyaluronsäure und Chondroitinsulfat in das Urothel eingebaut werden bzw. sich hieran anlagern, so daß ein durch entzündliche Reaktionen hervorgerufener Verlust von Hyaluronsäure und Chondroitinsulfat in der Blasenwand bzw. im Urothel sozusagen kompensiert wird. Es liegt somit gewissermaßen eine Regulation der Permeabilität bzw. Durchlässigkeit der Blasenwand vor, was zu einer Eindämmung der Entzündungsreaktion und somit zu einer Unterstützung der Wundheilung und damit insgesamt zu einer Verbesserung des Gesundheitszustandes führt. Die erfindungsgemäße Zubereitung bildet quasi einen Schutz des Blasenepithels vor irritierenden Substanzen, wie Bakterien, Mikrokristallen. Sie fungiert somit als Ersatz und Schutz der Glykosaminoglykanschicht in der Harnblase und den ableitenden Harnwegen.

Zu Zwecken der erfindungsgemäßen Verwendung wird die erfindungsgemäße Zubereitung - wie zuvor beschrieben - im Urogenitalbereich, insbesondere in die Harnblase instilliert bzw. vorzugsweise topisch appliziert. Dabei sollte die Instillation bzw. die Applikation in die vorzugsweise entleerte Harnblase erfolgen. Diesbezüglich sollte die Zubereitung nach der Erfindung in der Harnblase für einen Zeitraum von 30 bis 120 Minuten verbleiben, um eine optimale Einwirkung der Wirkstoffe auf das Urothel zu ermöglichen. Folglich sollte - genauer gesagt - das gesamte Volumen der in dem erfmdungsgemäßen Behältnis bzw. in der erfindungsgemäßen Applikationsvorrichtung vorhandenen Zubereitung nach der Erfindung, vorzugsweise 50 ml, nach vollständiger Entleerung der Harnblase in die Harnblase instilliert werden. Um optimale Ergebnisse zu erreichen, sollte die Zubereitung nach der Erfindung möglichst lange in der Harnblase verbleiben, und zwar - wie zuvor beschrieben - für einen Zeitraum von etwa 30 Minuten bis maximal etwa zwei Stunden. Im Rahmen der Behandlung der Cystitis ist es empfehlenswert, die Instillation der erfindungsgemäßen Zubereitung für einen Zeitraum von vier Wochen jeweils einmal pro Woche durchzuführen, wobei die diesbezügliche Dosis 50 ml pro Anwendung betragen sollte. Im Rahmen einer Erhaltungstherapie kann die erfindungsgemäße Zubereitung anschließend einmal pro Monat angewendet werden, bis die Symptome abgeklungen sind.

Mit anderen Worten kann vorzugsweise das gesamte Volumen der Lösung, bevorzugt 50 ml, aus dem Behältnis bzw. der Applikationsvorrichtung nach vollständiger Entleerung der Harnblase in die Harnblase instilliert werden. Um optimale Ergebnisse zu erreichen, sollte die erfindungsgemäße Zubereitung möglichst lange in der Harnblase verbleiben (mindestens etwa 30 Minuten, maximal etwa zwei Stunden). Besonders gute Ergebnisse wurden erreicht, wenn die Behandlung vier Wochen einmal pro Woche durchgeführt wurde. Als Erhaltungstherapie kann die Zubereitung nach der Erfindung einmal pro Monat angewendet werden, bis die Symptome vollständig abgeklungen sind. Von dem vorgenannten Therapieschema kann jedoch auch abgewichen werden, sofern einzelfallbezogen erforderlich.

Ein weiterer, großer Vorteil der erfindungsgemäßen Zubereitung ist darin zu sehen, daß sie hinsichtlich ihrer Anwendung sicher ist und während der Behandlung keine nennenswerten Nebenwirkungen auftreten, insbesondere da es sich bei den eingesetzten Substanzen um biokompatible Stoffe handelt. Die besonders gute Verträglichkeit steht insbesondere mit der Verwendung von Hyaluronsäure bzw, deren Salze nichttierischen Ursprungs und von Chondroitinsulfat marinen Ursprungs in Zusammenhang.

Zusammenfassend handelt es sich bei der erfindungsgemäßen Zubereitung vorzugsweise um ein Medizinprodukt, das gemäß einer erfindungsgemäß bevorzugten Ausführungsform eine Lösung von hochgereinigter Hyaluronsäure nichttierischen Ursprungs und Chondroitinsulfat marinen Ursprungs enthält. Es kann vorzugsweise als sterile Spülflüssigkeit bei Veränderungen des Urothels im Harnleiter, der Harnblase und dem Anfangsteil der Harnröhre eingesetzt werden. Wie zuvor erörtert, hat die erfindungsgemäße Zubereitung - ohne sich auf diese Theorie beschränken zu wollen - unmittelbar keinen pharmakologischen Effekt auf das Gewebe nach seiner Einbringung bzw. Instillation in die Harnblase. Seine Wirkung beruht auf physikalischen Eigenschaften. So kann ein Wirkmechanismus - ohne sich hierauf beschränken zu wollen - darin gesehen werden, daß der durch Entzündungen des Urothels verursachte Verlust von Hyaluronsäure und Chondroitinsulfat in der Blasenwand durch die Substitution von Hyaluronsäure und Chondroitinsulfat kompensiert wird und somit das Urothel regenerieren kann. Als Folge des Einsatzes der erfindungsgemäßen Zubereitung kommt es zu einer Regulation der Durchlässigkeit der Blasenwand, zu einer Eindämmung der Entzündungsreaktion und/oder zu einer Unterstützung der Wundheilung.

Die erfindungsgemäße Zubereitung enthält vorzugsweise eine sterile Hyaluronsäure/Chondroitinsulfat-Lösung, die beispielsweise mit Hilfe eines Katheters in die Harnblase eingespült bzw. instilliert werden kann. Die erfindungsgemäße Zubereitung insbesondere in Form einer Lösung und/oder Spülflüssigkeit dient der Regeneration des gestörten bzw. veränderten Urothels in der Harnblase und den ableitenden Harnwegen.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

### Ausführungsbeispiele:

Im Rahmen von seitens der Anmelderin durchgeführten Ausführungsbeispielen bzw. Wirksamkeitsstudien wurde die herausragende Wirkung einer Zubereitung nach der vorliegenden Erfindung auf Basis der erfindungsgemäßen Wirkstoffkombination (vgl. nachfolgende "Zubereitung F") gegenübe verschiedenen Vergleichszubereitungen (vgl. nachfolgende "Zubereitungen A bis E") und einer Blindprobe ("Placebo") belegt. In diesem Zusammenhang wurde sowohl die verbesserte Wirksamkeit der erfindungsgemäß verwendeten Einzelkomponenten - nämlich Hyaluronsäure nichttierischen Ursprungs mit einer mittleren Molmasse von weniger als 200 kDa einerseits und Chondroitinsulfat marinen Ursprungs andererseits - als auch der durch die Kombination dieser beiden Einzelsubstanzen erhaltene synergistische Effekt belegt.

Die hervorragende Wirksamkeit der erfindungsgemäßen Kombination wurde anhand von drei verschiedenen Untersuchungsmethoden (d. h. subjektives Wohlbefinden der Patienten, Cystoskopie und Glykosaminoglykangehalt im Urin) ermittelt. Alle Zubereitungen sind auf Basis einer Natriumchloridlösung formuliert und enthalten die folgenden Wirkstoffe:

### Zubereitung A (Vergleich):

| | |
|---|---|
| Natriumhyaluronat tierischen Ursprungs (M > 200 kDa) | 40 mg / 50 ml |

### Zubereitung B (Vergleich):

| | |
|---|---|
| Chondroitinsulfat nichtmarinen Ursprungs | 40 mg / 50 ml |

### Zubereitung C (Vergleich):

| | |
|---|---|
| Natriumhyaluronat tierischen Ursprungs (M > 200 kDa) | 40 mg / 50 ml |
| Chondroltinsulfat nichtmarinen Ursprungs | 40 mg / 50 ml |

### Zubereitung D (Vergleich):

| | |
|---|---|
| Natriumhyaluronat nichttierischen Ursprungs (M=180 kDa) | 40 mg / 50 ml |

### Zubereitung E (Vergleich):

| | |
|---|---|
| Chondroitinsulfat marinen Ursprungs | 40 mg / 50 ml |

### Zubereitung F (Erfindung):

| | |
|---|---|
| Natriumhyaluronat nichttierischen Ursprungs (M=180 kDa) | 40 mg / 50 ml |
| Chondroitinsulfat marinen Ursprungs | 40 mg / 50 ml |

### Zubereitung G (placebo): reine Natriumchloridlösung (Osmolarität entsprechend der von Urin).

Was die erfindungsgemäße Zubereitung F anbelangt, so wurde in bezug auf das Chondroitinsulfat marinen Ursprungs eine Mischung von Chondroitin-6-sulfat und Chondroitin-4-sulfat im Verhältnis von etwa 80 bis 90 Gew.-% Chondroitin-6-sulfat zu etwa 20 bis 10 Gew.-% Chondroitin-4-sulfat eingesetzt.

Im Rahmen der vorgenannten Zubereitungen bzw. Ansätze wurde jeweils das Natriumsalz von Hyaluronsäure bzw. Chondroitinsulfat verwendet.

Probanden mit einer diagnostizierten interstitiellen (d. h. nichtbakteriellen) Cystis wurde über einen Zeitraum von zwei Monaten die oben angeführten Zubereitungen verabreicht. Die Verabreichung erfolgte mittels Instillation in die Harnblase. Die Werte zum Zeitpunkt t = 0 beziehen sich auf den Zeitpunkt unmittelbar vor der ersten Verabreichung. Die Verabreichung wurde jeweils über einen Zeitraum von zwei Monaten wöchentlich wiederholt. Das Volumen der instillierten Zubereitung betrug jeweils 50 ml. Pro Ansatz bzw. Zubereitung wurden 10 Probanden untersucht. Die Untersuchungsabstände betrugen jeweils 1 Woche, 2 Wochen, 1 Monat und schließlich 2 Monate, jeweils bezogen auf den Tag der Erstinstillation.
1. Im Rahmen des ersten Untersuchungskomplexes gaben die Probanden ihr subjektives Empfinden anhand einer Schulnotenskala von 1 bis 6 (1= sehr gut bis 6 = ungenügend) an, wobei auch beliebige Zwischenwerte möglich waren. Bei dieser Untersuchung wurde insbesondere auf Schmerzen im Bereich der Harnblase sowie des Beckens, übermäßigen Harndrang und auf eine kleine Harnblasenkapazität abgestellt. Es wurden die jeweiligen Mittelwerte sowie die dazugehörigen Standardabweichungen ermittelt. Tabelle 1 zeigt die ermittelten Ergebnisse. Auffällig ist, daß bei einem Vergleich der Einzelsubstanzen gemäß Zubereitung A und B einerseits sowie Zubereitung C und D andererseits die verwendeten Substanzen gemäß Zubereitungen C und D zu besseren Ergebnissen führen als die Substanzen tierischen Ursprungs.
Was die erfindungsgemäße Kombination gemäß Zubereitung F anbelangt, so war eine deutliche Verbesserung des gesundheitlichen Wohlbefindens zu beobachten, so daß nach Ende des Versuchszeitraums die untersuchten Probanden insgesamt über ein "gutes" gesundheitliches Wohlbefinden berichteten.

**Tabelle 1**

| | **A (Vgl.)** | **B (Vgl.)** | **C (Vgl.)** | **D (Vgl.)** | **E (Vgl.)** | **F (erf.)** | **G (Plac.)** |
|---|---|---|---|---|---|---|---|
| t=0 | 5,2±0,3 | 5,0±0,2 | 4,8±0,1 | 5,3±0,2 | 4,9±0,4 | 4,9±0,3 | 5,5±0,3 |
| 1. Woche | 5,1±0,4 | 5.0±0,3 | 3,8±0,1 | 4,6±0,2 | 4.8±0,4 | 3,5±0,1 | 5,5±0,4 |
| 2.Woche | 4,6±0,3 | 4,8±0,4 | 3,6±0,3 | 4,6±0,5 | 4,5±0,1 | 3,0±0,3 | 5,3±0,5 |
| 1. Monat | 4,0±0,2 | 4,3±0,3 | 3,1±0,3 | 3,3±0,3 | 3,5±0,2 | 2,4±0,2 | 5,9±0,4 |
| 2.Monat | 3,6±0,3 | 4,1±0,3 | 3,8±0,2 | 3,1±0,3 | 3,3±0,2 | 2,0±0,4 | 4.8±0,5 |

2. In einer zweiten Untersuchung wurde einen Monat nach der ersten Applikation der vorgenannten Zubereitungen bei den jeweiligen Probandengruppen eine Cytoskopie (Blasenspiegelung) durchgeführt, wobei mittels Endoskop eine visuelle Begutachtung des Blasenurothels durchgeführt wurde. Der visuelle Befund ist in Tabelle 2 in bezug auf die jeweiligen Zubereitungen angegeben. Auch hier zeigt sich die deutlich verbesserte Wirkung der erfindungsgemäßen Zubereitung auf Basis der binären Kombination von nichttierischem Hyaluronat mit einer Molmasse von weniger als 200 kDa einerseits und marinern Chondroitinsulfat andererseits gegenüber den Vergleichszubereitungen

**Tabelle 2**

| **A (Vgl.)** | **B (Vgl.)** | **C (Vgl.)** | **D (Vgl.)** |
|---|---|---|---|
| Mäßige bis starke Defekte des Blasenmucus, teilweise Läsionen und Verkrustungen, geringe Einblutungen | Mäßige bis starke Defekte des Blasenmucus, teilweise Läsionen und Verkrustungen, geringe Einblutungen | Mäßige Defekte des Biesenmucus, geringfügige Läsionen, keine Einblutungen | Mäßige bis schwache Läsionen des Blasenmucus, leichte bis mittlere Läsionen, kaum Einblutungen |

| **E (Vgl.)** | **F (erf.)** | **G (Plac.)** | |
|---|---|---|---|
| Mäßiger bis geringer Defekt des Blasenmucus, kaum Läsionen, wenige Einblutungen | Nahezu intakter Blasenmucus, keine Läsionen, keine Einblutungen | Starke Defekte des Blasenmucus, Starke Läsionen mit teilweise mäßigen bis starken Einblutungen | |

3. Darüber hinaus wurden zum Versuchsbeginn sowie nach einem Monat Urinproben genommen und der Gehalt an Glykosaminoglykan im Urin in Anlehnung an das Verfahren nach *Whitley et al.* (vgl. C.*B.,* Ridnour, M. D., Draper, K A., Dutton, C. M. and Negila, J. P.: Diagnostic test for mucoplysaccharidosis. I. Direct method for quantifying excessive urinary glycosaminoglycan excretion. Clin. Chem., 35: 374, 1989) ermittelt, wobei die im Urin vorhandenen Glykosaminoglykane mittels Dimethylmethylenblau angefärbt und deren Konzentrationen anschließend spektroskopisch bestimmt wurden. In diesem Zusammenhang ist bekannt, daß bei Patienten mit diagnostizierter interstitieller Cystitis eine von der Konzentration bei gesunden Patienten, d. h. bei Patienten ohne Befund einer interstitiellen Cystitis, abweichende Konzentration an im Urin vorhandenem Glykosaminoglykan vorliegt. Diesbezüglich ist auch bekannt, daß bei Patienten mit einer weit fortgeschrittenen bzw. chronischen interstitiellen Cystitis der Gehalt an Glykosaminoglykan im Urin im Vergleich zu einer Kontrollgruppe ohne Befund verringert ist, während der Gehalt an Glykosaminoglykan im Urin bei Probanden mit interstitieller Cystitis im Anfangsstadium bzw. im nicht weit fortgeschrittenen Stadium erhöht ist.
Zu Versuchsbeginn (t = 0) wurden Urinproben hinsichtlich ihres Glykosaminoglykangehaltes analysiert, wobei in sämtlichen Gruppen (d.h. bei Probanden, denen nachfolgend mehrmalig eine der Zubereitungen A bis G instilliert wurde) im Vergleich zu einer Kontrollgruppe ohne interstitielle Cystitis abweichende Konzentrationen an Glykosaminoglykan im Urin festgestellt wurden.
Nach einem Monat wurden erneut Urinproben analysiert. Für die Placebogruppe G trat keine Normalisierung der ermittelten Glykosaminoglykangehalte auf, teilweise verschlechterten sich die Werte sogar noch. In bezug auf die Zubereitungen A und B wurde nur eine geringfügige Verbesserung des Glykosaminoglykangehaltes festgestellt. In einigen Fällen war die Abweichung vom Normwert nach wie vor erheblich. In bezug auf die Zubereitung C konnte zwar eine deutlichere Normalisierung festgestellt werden, jedoch waren auch hier noch deutliche Abweichungen vorn Normwert festzustellen. Was die Was die Zubereitungen D und E anbelangt, so waren die Werte insbesondere im Vergleich zu den Zubereitungen A und B etwas verbessert, jedoch trat auch hier keine vollständige Normalisierung auf. Was die erfindungsgemäße Kombination gemäß der Zubereitung F anbelangt, so konnte hier eine signifikante Verbesserung festgestellt werden: Bei nahezu allen Probanden trat eine Normalisierung des Glykosaminoglykangehaltes im Urin ein, die Werte entsprachen nahezu vollständig den für die Kontrollgruppe ohne Befund einer interstitiellen Cystitis ermittelten Werten.
Auch dieser Test belegt somit die besonders gute Wirkung der erfindungsgemäßen Kombination gemäß Zubereitung F im Vergleich zur Kontrolluntersuchung (Placebo) gemäß Versuchsgruppe G sowie gegenüber den anderen Vergleichs Zubereitungen A bis E.

Die vorstehenden Versuchsreihen zeigen insgesamt die hervorragende, synergistische Wirkung der erfindungsgemäßen binären Kombination gegenüber den jeweiligen Kontrollexperimenten.

## Patentansprüche

1. Zubereitung, insbesondere zur Behandlung von entzündlichen Erkrankungen des Urogenitaltraktes, vorzugsweise von Cystitis, enthaltend in Kombination und jeweils in pharmazeutisch wirksamen Mengen
(a) Hyaluronsäure und/oder deren Salze mit einer mittleren Molmasse (Molekulargewicht) von weniger als 200 kDa und
(b) mindestens ein Chondroitinsulfat.

2. Zubereitung nach Anspruch 1, wobei (a) die Hyaluronsäure und/ oder deren Salze eine mittlere Molmasse im Bereich von 50 bis 195 kDa, insbesondere 100 bis 195 kDa, vorzugsweise 150 bis 190 kDa, bevorzugt 175 bis 190 kDa, aufweist

3. Zubereitung nach Anspruch 1 oder 2, enthaltend (a) Hyaluronsäure und/oder deren Salze in Mengen von 0,02 bis 0,2 Gew.-%, insbesondere 0,03 bis 0,16 Gew.-%, vorzugsweise 0,04 bis 0,12 Gew.-%, bevorzugt 0,05 bis 0,10 Gew.-%, bezogen auf das Gesamtgewicht der Zubereitung.

4. Zubereitung nach einem oder mehreren der vorangehenden Anspruche, wobei (a) die Hyaluronsäure und/oder deren Salze nichttierischen Ursprungs ist und/oder wobei die Hyaluronsäure und/oder deren Salze bakteriellen Ursprungs ist, insbesondere wobei die Hyaluronsäure und/oder deren Salze vorzugsweise fermentativ von Bakterien der Gattung *Streptococcus,* insbesondere *Streptococcus lancefields,* bevorzugt *Streptococcus lancefields* Stamm A, gewonnen ist.

5. Zubereitung nach einem oder mehreren der vorangehenden Ansprüche, wobei (b) das Chondroitinsulfat marinen Ursprungs ist und/oder wobei (b) das Chondroitinsulfat eine mittlere Molmasse im Bereich von 10 bis 70 kDa, insbesondere 20 bis 60 kDa, vorzugsweise 25 bis 50 kDa, bevorzugt 30 bis 40 kDa, aufweist,

6. Zubereitung nach einem oder mehreren der vorangehenden Ansprüche, enthaltend (b) das Chondroitinsulfat in Mengen von 0,02 bis 0,2 Gew.-%, insbesondere 0,03 bis 0,16 Gew.-%, vorzugsweise 0,04 bis 0,12 Gew.-%, bevorzugt 0,05 bis 0,10 Gew.-%, bezogen auf das Gesamtgewicht der Zubereitung.

7. Zubereitung nach einem oder mehreren der vorangehenden Ansprüche, wobei das Mengenverhältnis (Gewichtsverhältnis) von (a) Hyaluronsäure und/oder deren Salzen einerseits zu (b) Chondroitinsulfat andererseits in der Zubereitung im Bereich von 0,25 : 2 bis 2 : 0,25, insbesondere 0,5 : 1,5 bis 1,5 : 0,5, vorzugsweise 0,75: 1,25 bis 1,25 :0,75, besonders bevorzugt etwa 1 : 1, liegt.

8. Zubereitung nach einem oder mehreren der vorangehenden Ansprüche, wobei die Zubereitung in flüssiger Form, insbesondere in Form einer wäßrigen Lösung oder einer wäßrigen Suspension, vorliegt.

9. Zubereitung nach einem oder mehreren der vorangehenden Ansprüche, wobei die Zusammensetzung eine Osmolarität von 50 bis 1000 mOsmol/l, insbesondere 100 bis 800 mOsmol/l, vorzugsweise 150 bis 600 mOsmol/l, bevorzugt 200 bis 400 mOsmol/l, aufweist und/oder wobei die Zubereitung einen pH-Wert von 6 bis 8, insbesondere 6,5 bis 7,8, vorzugsweise 7 bis 7,5, aufweist und/oder wobei die Zubereitung bei einer Temperatur von 20 °C eine dynamische Viskosität von 5.000 bis 12.000 mPas, insbesondere 6.000 bis 10.000 mPas, vorzugsweise 7.500 bis 9.500 mPas, bevorzugt 8.000 bis 9.000 mPas, aufweist.

10. Zubereitung nach einem oder mehreren der vorangehenden Ansprüche, insbesondere in Form einer wäßrigen Lösung oder einer wäßrigen Suspension, enthaltend in Kombination
(a) Hyaluronsäure und/oder deren Salze, insbesondere nichttierischen Ursprungs, vorzugsweise Natriumhyaluronat, bevorzugt Natriumhyaluronat nichttierischen Ursprungs, mit einer mittleren Molmasse (Molekulargewicht) von weniger als 200 kDa, insbesondere mit einer Molmasse im Bereich von 50 bis 195 kDa, insbesondere 100 bis 195 kDa, vorzugsweise 150 bis 190 kDa, bevorzugt 175 bis 190 kDa, und/oder insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml besonders bevorzugt 0,7 bis 0,9 mg/ml, bezogen auf das Volumen der Zubereitung,
(b) mindestens ein Chondroitinsulfat, insbesondere marinen Ursprungs, insbesondere mit einem Wirkstoffgehalt von 0,2 bis 2,0 mg/ml, insbesondere 0,3 bis 1,6 mg/ml, vorzugsweise 0,4 bis 1,2 mg/ml, bevorzugt 0,5 bis 1,0 mg/ml, besonders bevorzugt 0,7 bis 0,9 mg/ml, und
(c) gegebenenfalls mindestens einen Elektrolyten, vorzugsweise Natriumchlorid, insbesondere mit einem Gehalt von 0,05 bis 0,3 mg/ml, insbesondere 0,07 bis 0,2 mg/ml, vorzugsweise 0,1 bis 0,17 mg/ml,
insbesondere wobei das Mengenverhältnis (Gewichtsverhältnis) von (a) Hyaluronsäure und/oder deren Salzen, vorzugsweie Natriumhyaluronat, einerseits zu (b) Chondroitinsulfat andererseits in der Zubereitung im Bereich von 0,25 : 2 bis 2 : 0,25, insbesondere 0,5 : 1,5 bis 1,5 : 0,5, vorzugsweise 0,75 : 1,25 bis 1,25 : 0,75, besonders bevorzugt etwa 1 : 1, liegt.

11. Zubereitung nach einem oder mehreren der vorangehenden Ansprüche zur Instillation und/oder zur vorzugsweise topischen Applikation in den Urogenitalbereich, insbesondere in die Harnblase.

12. Zubereitung nach einem oder mehreren der vorangehenden Ansprüche zur prophylaktischen und/oder therapeutischen Behandlung von Cystitis, insbesondere akuter oder chronischer Cystitis, vorzugsweise interstitieller Cystitis, radiogener Cystitis und/oder chronisch (chronifizierter) bakterieller Cystitis, bevorzugt interstitieller Cystitis.

13. Behältnis, enthaltend eine Zubereitung nach einem oder mehreren der vorangehenden Ansprüche, insbesondere in Form einer Durchstichflasche mit vorzugsweise sterilem Verschluß.

14. Applikationsvorrichtung zur Instillation und/oder zur vorzugsweise topischen Applikation in den Urogenitalbereich, insbesondere in die Harnblase, enthaltend eine Zubereitung nach einem der Ansprüche 1 bis 12.

15. Verwendung einer Zubereitung nach einem der Ansprüche 1 bis 12 zur Herstellung eines Arzneimittels zur Behandlung von Erkrankungen des Urogenitaltraktes, insbesondere von entzündlichen Erkrankungen der Harnblase, vorzugsweise von Cystitis, bevorzugt von interstitieller Cystitis, radiogener Cystitis und/oder chronifizierter bakterieller Cystitis, besonders bevorzugt von interstitieller Cystitis.

## Claims

1. Preparation, particularly for treating inflammatory diseases of the urogenital tract, preferably for treating cystitis, containing a combination of the following, each in pharmaceutically active amounts:
(a) hyaluronic acid and/or salts thereof having a mean molar mass (molecular weight) of less than 200 kDa, and
(b) at least one chondroitin sulphate.

2. Preparation according to Claim 1, wherein (a) the hyaluronic acid and/or salts thereof has/have a mean molar mass in the range of 50 to 195 kDa, particularly 100 to 195 kDa, preferably 150 to 190 kDa, more preferably 175 to 190 kDa.

3. Preparation according to Claim 1 or 2, containing (a) hyaluronic acid and/or salts thereof in amounts of 0.02 to 0.2% by weight, particularly 0,03 to 0.16% by weight, preferably 0.04 to 0.12% by weight, more preferably 0.05 to 0.10% by weight, relative to the total weight of the preparation.

4. Preparation according to one or more of the preceding claims, wherein (a) the hyaluronic acid and/or salts thereof is/are of non-animal origin, and/or wherein the hyaluronic acid and/or salts thereof is/are of bacterial origin, particularly wherein the hyaluronic acid and/or salts thereof is/are recovered preferably by fermentation from bacteria of the genus *Streptococcus,* particularly *Streptococcus lancefields,* preferably *Streptococcus lancefields* strain A.

5. Preparation according to one or more of the preceding claims, wherein (b) the chondroitin sulphate is of marine origin and/or wherein (b) the chondroitin sulphate has a mean molar mass in the range of 10 to 70 kDa, particularly 20 to 60 kDa, preferably 25 to 50 kDa, more preferably 30 to 40 kDa.

6. Preparation according to one or more of the preceding claims, containing (b) the chondroitin sulphate in amounts of 0.02 to 0.2% by weight, particularly 0.03 to 0.16% by weight, preferably 0.04 to 0.12% by weight, more preferably 0.05 to 0.10% by weight, relative to the total weight of the preparation.

7. Preparation according to one or more of the preceding claims, wherein the mass ratio (weight ratio) of (a) hyaluronic acid and/or salts thereof, on the one hand, to (b) chondroitin sulphate, on the other hand, in the preparation is in the range of 0.25:2 to 2:0.25, particularly 0.5:1.5 to 1.5:0.5, preferably 0.75:1.25 to 1.25:0.75, particularly preferably about 1:1.

8. Preparation according to one or more of the preceding claims, wherein the preparation is present in liquid form, particularly in the form of an aqueous solution or an aqueous suspension.

9. Preparation according to one or more of the preceding claims, wherein the composition has an osmolarity of 50 to 1000 mOsmol/l, particularly 100 to 800 mOsmol/l, preferably 150 to 600 mOsmol/l, more preferably 200 to 400 mOsmol/l, and/or wherein the preparation has a pH value of 6 to 8, particularly 6.5 to 7.8, preferably 7 to 7.5, and/or wherein the preparation at a temperature of 20°C has a dynamic viscosity of 5000 to 12000 mPas, particularly 6000 to 10000 mPas, preferably 7500 to 9500 mPas, more preferably 8000 to 9000 mPas.

10. Preparation according to one or more of the preceding claims, particularly in the form of an aqueous solution or an aqueous suspension, containing in combination:
(a) hyaluronic acid and/or salts thereof, particularly of non-animal origin, preferably sodium hyaluronate, more preferably sodium hyaluronate of non-animal origin, with a mean molar mass (molecular weight) of less than 200 kDa, particularly with a molar mass in the range of 50 to 195 kDa, particularly 100 to 195 kDa, preferably 150 to 190 kDa, more preferably 175 to 190 kDa, and/or particularly with an active substance content of 0.2 to 2.0 mg/ml, particularly 0.3 to 1.6 mg/ml, preferably 0.4 to 1.2 mg/ml, more preferably 0.5 to 1.0 mg/ml, particularly preferably 0.7 to 0.9 mg/ml, relative to the volume of the preparation,
(b) at least one chondroitin sulphate, particularly of marine origin, particularly with an active substance content of 0.2 to 2.0 mg/ml, particularly 0.3 to 1.6 mg/ml, preferably 0.4 to 1.2 mg/ml, more preferably 0.5 to 1.0 mg/ml, particularly preferably 0.7 to 0.9 mg/ml, and
(c) optionally at least one electrolyte, preferably sodium chloride, particularly with a content of 0.05 to 0.3 mg/ml, particularly 0.07 to 0.2 mg/ml, preferably 0.1 to 0.17 mg/ml,
particularly wherein the mass ratio (weight ratio) of (a) hyaluronic acid and/or salts thereof, preferably sodium hyaluronate, on the one hand to (b) chondroitin on the other hand in the preparation, is in the range of 0.25:2 to 2:0.25, particularly 0.5:1.5 to 1.5:0.5, preferably 0.75:1.25 to 1.25:0.75, particularly preferably about 1:1.

11. Preparation according to one or more of the preceding claims, for instillation and/or for preferably topical application into the urogenital area, particularly into the bladder.

12. Preparation according to one or more of the preceding claims for prophylactic and/or therapeutic treatment of cystitis, particularly of acute or chronic cystitis, preferably interstitial cystitis, radiogenic cystitis and/or chronic (chronically infected) bacterial cystitis, more preferably interstitial cystitis.

13. Container containing a preparation according to one or more of the preceding claims, particularly in the form of a pierceable vial with a preferably sterile closure.

14. Applicator device for instillation and/or for preferably topical application into the urogenital area, particularly into the bladder, containing a preparation according to one of Claims 1 to 12.

15. Use of a preparation according to one of Claims 1 to 12 for producing a medicament for treating diseases of the urogenital tract, particularly inflammatory diseases of the bladder, preferably cystitis, more preferably interstitial cystitis, radiogenic cystitis and/or chronic bacterial cystitis, particularly preferably interstitial cystitis.

## Revendications

1. Préparation, en particulier pour le traitement de maladies inflammatoires du tractus urogénital, de préférence de la cystite, contenant en combinaison et à chaque fois en des quantités pharmaceutiquement actives
(a) de l'acide hyaluronique et/ou ses sels avec une masse molaire moyenne (poids moléculaire) inférieure à 200 kDa, et
(b) au moins un sulfate de chondroïtine.

2. Préparation selon la revendication 1, où (a) l'acide hyaluronique et/ou ses sels présente(nt) une masse molaire moyenne dans la plage de 50 à 195 kDa, en particulier de 100 à 195 kDa, de préférence de 150 à 190 kDa, préférablement de 175 à 190 kDa.

3. Préparation selon la revendication 1 ou 2, contenant (a) de l'acide hyaluronique et/ou ses sels en des quantités de 0,02 à 0,2% en poids, en particulier de 0,03 à 0,16% en poids, de préférence de 0,04 à 0,12% en poids, préférablement de 0,05 à 0,10% en poids, par rapport au poids total de la préparation.

4. Préparation selon l'une ou plusieurs des revendications précédentes, où (a) l'acide hyaluronique et/ou ses sels est/sont d'origine non animale et/ou où l'acide hyaluronique et/ou ses sels est/sont d'origine bactérienne, en particulier où l'acide hyaluronique et/ou ses sels est/sont de préférence produit(s) par fermentation de bactéries du type Streptococcus, en particulier Streptococcus lancefields, de préférence Streptococcus lancefields souche A.

5. Préparation selon l'une ou plusieurs des revendications précédentes, où (b) le sulfate de chondroïtine est d'origine marine et/ou où (b) le sulfate de chondroïtine présente une masse molaire moyenne dans la plage de 10 à 70 kDa, en particulier de 20 à 60 kDa, de préférence de 25 à 50 kDa, préférablement de 30 à 40 kDa.

6. Préparation selon l'une ou plusieurs des revendications précédentes, contenant (b) le sulfate de chondroïtine en des quantités de 0,02 à 0,2% en poids, en particulier de 0,03 à 0,16% en poids, de préférence de 0,04 à 0,12% en poids, préférablement de 0,05 à 0,10% en poids, par rapport au poids total de la préparation.

7. Préparation selon l'une ou plusieurs des revendications précédentes, où le rapport de quantités (rapport pondéral) de (a), l'acide hyaluronique et/ou ses sels d'une part, à (b), le sulfate de chondroïtine d'autre part, dans la préparation se situe dans la plage de 0,25:2 à 2:0,25, en particulier de 0,5:1,5 à 1,5:0,5, de préférence de 0,75:1,25 à 1,25:0,75, de manière particulièrement préférée à environ 1:1.

8. Préparation selon l'une ou plusieurs des revendications précédentes, où la préparation se trouve sous forme liquide, en particulier sous forme d'une solution aqueuse ou d'une suspension aqueuse.

9. Préparation selon l'une ou plusieurs des revendications précédentes, où la composition présente une osmolarité de 50 à 1000 mOsmoles/l, en particulier de 100 à 800 mOsmoles/l, de préférence de 150 à 600 mOsmoles/l, préférablement de 200 à 400 mOsmoles/l, et/ou où la préparation présente un pH de 6 à 8, en particulier de 6,5 à 7,8, de préférence de 7 à 7,5, et/ou où la préparation présente, à une température de 20°C, une viscosité dynamique de 5000 à 12 000 mPas, en particulier de 6000 à 10 000 mPas, de préférence de 7500 à 9500 mPas, préférablement de 8000 à 9000 mPas.

10. Préparation selon l'une ou plusieurs des revendications précédentes, en particulier sous forme d'une solution aqueuse ou d'une suspension aqueuse, contenant en combinaison
(a) de l'acide hyaluronique et/ou ses sels, en particulier d'origine non animale, de préférence l'hyaluronate de sodium, de préférence l'hyaluronate de sodium d'origine non animale, présentant une masse molaire moyenne (poids moléculaire) inférieure à 200 kDa, en particulier présentant une masse molaire dans la plage de 50 à 195 kDa, en particulier de 100 à 195 kDa, de préférence de 150 à 190 kDa, préférablement de 175 à 190 kDa, et/ou en particulier présentant une teneur en substance active de 0,2 à 2,0 mg/ml, en particulier de 0,3 à 1,6 mg/ml, de préférence de 0,4 à 1,2 mg/ml, préférablement de 0,5 à 1,0 mg/ml, de manière particulièrement préférée de 0,7 à 0,9 mg/ml, par rapport au volume de la préparation,
(b) au moins un sulfate de chondroïtine, en particulier d'origine marine, présentant en particulier une teneur en substance active de 0,2 à 2,0 mg/ml, en particulier de 0,3 à 1,6 mg/ml, de préférence de 0,4 à 1,2 mg/ml, préférablement de 0,5 à 1,0 mg/ml, de manière particulièrement préférée de 0,7 à 0,9 mg/ml, et
(c) le cas échéant au moins un électrolyte, de préférence le chlorure de sodium, en particulier en une teneur de 0,05 à 0,3 mg/ml, en particulier de 0,07 à 0,2 mg/ml, de préférence de 0,1 à 0,17 mg/ml,
en particulier où le rapport des quantités (rapport pondéral) de (a) acide hyaluronique et/ou ses sels, de préférence l'hyaluronate de sodium d'une part, à (b) sulfate de chondroïtine d'autre part, dans la préparation se situe dans la plage de 0,25:2 à 2:0,25, en particulier de 0,5:1,5 à 1,5:0,5, de préférence de 0,75:1,25 à 1,25:0,75, de manière particulièrement préférée à environ 1:1.

11. Préparation selon l'une ou plusieurs des revendications précédentes destinée à l'instillation et/ou à l'application de préférence topique dans le domaine urogénital, en particulier dans la vessie.

12. Préparation selon l'une ou plusieurs des revendications précédentes pour le traitement prophylactique et/ou thérapeutique de la cystite, en particulier la cystite aiguë ou chronique, de préférence la cystite interstitielle, la cystite radiogène et/ou la cystite bactérienne chronique (chronicisée), de préférence la cystite interstitielle.

13. Récipient contenant une préparation selon l'une ou plusieurs des revendications précédentes, en particulier sous forme d'un flacon à perforer avec une fermeture de préférence stérile.

14. Dispositif d'application destiné à l'instillation et/ou à l'application de préférence topique dans le domaine urogénital, en particulier dans la vessie, contenant une préparation selon l'une quelconque des revendications 1 à 12.

15. Utilisation d'une préparation selon l'une quelconque des revendications 1 à 12 pour la préparation d'un médicament destiné au traitement de maladies du tractus urogénital, en particulier de maladies inflammatoires de la vessie, de préférence de la cystite, préférablement de la cystite interstitielle, de la cystite radiogène et/ou de la cystite bactérienne chronicisée, de manière particulièrement préférée de la cystite interstitielle.
